# EUROPEAN PATENT SPECIFICATION

(11) **EP 4 432 861 B1**
(45) Date of publication and mention of the grant of the patent: **28.01.2026**
(21) Application number: 22818325.7
(22) Date of filing: 16.11.2022
(51) Int. Cl.: A23P 10/25, A23P 30/20

(54) **GRANULE COMPRISING ENCAPSULATED ACTIVE INGREDIENTS**
GRANULAT, DAS EINGEKAPSELTE FETTLÖSLICHE WIRKSTOFFE ENTHÄLT
GRANULÉ COMPRENANT DES INGRÉDIENTS ACTIFS LIPOSOLUBLES ENCAPSULÉS

(30) Priority: 16.11.2021 EP 21208533
(43) Date of publication of application: 25.09.2024
(73) Proprietor: DSM IP Assets B.V., 6221 BE Maastricht (NL)
(72) Inventor: BEKAERT, Bram, 4303 Kaiseraugst (CH); PORTIER, Christoph, 4303 Kaiseraugst (CH); VANDEVIVERE, Lise, 4303 Kaiseraugst (CH); VERVAET, Chris, 4303 Kaiseraugst (CH)
(74) Representative: dsm-firmenich IP
(86) International application number: PCT/EP2022/082128
(87) International publication number: WO 2023/088962

(56) References cited:
- EP-A1- 1 972 202
- WO-A1-2004/043165
- WO-A1-2006/122021
- CN-A- 107 823 150
- CN-A- 113 598 397
- US-B1- 6 482 433

## Description

### Technical field

The present invention relates to water-dispersible powders for human consumption.

### Background of the invention

Fat-soluble active ingredients as such cannot be dissolved in water. It is therefore common practice to encapsulate fat-soluble active ingredients. Water-dispersible microcapsules comprising fat-soluble micronutrients (e.g. fat-soluble vitamins) are commercially available at DSM Nutritional Products (Switzerland).

Microcapsules are often poorly flowable, mostly due to their small particle size. To obtain a flowable powder, microcapsules are granulated.

Granulation is a size enlargement process that is often done via wet granulation using a solvent (water or organic solvent) to initiate binding between solid particles (e.g. microcapsules). Dry granulation and melt granulation are known alternatives for wet granulation. Melt granulation operates via similar principles as wet granulation but typically uses a molten binder as a granulation fluid to establish liquid bridges between the particles to be granulated. When cooling to room temperature, the binder solidifies and forms bridges between individual powder particles to yield a solid end product with a granular structure.

Most often, melt granulation is done in a heated powder bed. This is a batch process: processing of subsequent batches must wait until the current is finished. This also applies to wet granulation done in conventional high-shear mixers.

Documents CN-107823150A and WO-2004/043165A1 show compositions comprising microcapsules. Document WO-2006/122021A1 shows melt-extruded granules.

A drawback of wet granulation is the need of getting rid of the solvent at the end of the granulation process. In case of using water as solvent, a significant amount of energy is needed to evaporate water. A further drawback of wet granulation is the risk of hydrolysis of the active ingredient. In case of organic solvents, potentially harmful residues and/or negative environmental impacts are of concern.

During granulation, microcapsules and their content should remain essentially undamaged. The obtained granules should be flowable, storage stable and/or water-dispersible. The amount of fines (i.e. non-granulated residues) and/or the amount of surface oil should be low.

There is a need for a process for granulating microcapsules in a cost effective, efficient, and environmentally friendly manner.

### Summary of the invention

The problems underlying the present invention are solved by continuous melt granulation of a mixture that comprises microcapsules. The continuous melt granulation process is preferably done in an extruder. In a preferred embodiment of the invention, a co-rotating twin-screw extruder continuously churns out free flowing granules; no die is needed at the end of the extruder.

In addition to microcapsules, the mixture of the invention comprises at least one edible binder. During continuous melt granulation, the binder of the mixture is molten or at least softened.

In an embodiment, the microcapsules to be granulated comprise fat-soluble active ingredients. Some fat-soluble active ingredients are temperature sensitive. Prohibitively high temperature should be avoided when running the continuous melt granulation process of the invention. Thus, binders having a very high melting temperature or a very high glass transition (Tg) temperature are not preferred. In this context, "very high" can mean higher than 200°C or higher than 250° C.

In an embodiment, the mixture of invention further comprises at least one edible filler. In contrast to hot-melt extrusion, there is no need to melt the at least one edible filler during the continuous melt granulation process of the invention. The melting temperature of the at least one edible filler may therefore be significantly higher than the melting temperature of the at least one binder.

A preferred mixture comprises a filler, a binder and microcapsules,
wherein the mixture comprises from 0.1 weight-% to 55 weight-% of microcapsules, based on the total weight of the mixture, and
wherein the mixture comprises from 5 weight-% to 15 weight-% binder, based on the total weight of the mixture, and
wherein the weight ratio between filler and binder is from 4:1 to 9:1, and
wherein the melting temperature of the binder is lower than the melting temperature of the filler, and
wherein the melting temperature of the filler is from 151°C to 240°C.

In an embodiment, a method of manufacturing granules by continuous melt granulation comprises the step of feeding the mixture into an extruder, preferably into a twin-screw extruder. In an embodiment, the granules of the invention comprise the mixture of the invention. In an embodiment, the granules of the invention consist of the mixture of the invention.

### Detailed description of the invention

Continuous granulation is often done with extruders. Extrusion granulation can be done with a solvent ("continuous wet granulation") or with heat ("continuous melt granulation").

The granules of the invention are obtainable by continuous melt granulation of a dry, edible mixture that comprises primary particles and at least two edible excipients. During continuous melt granulation, primary particles are agglomerated. Thus, the granule of the invention is preferably a unit formed of numerous particles. Primary particles of a granule are smaller than the granule.

Both edible excipients are preferably water-soluble or water-dispersible. The melting temperature of a first edible excipient is low enough to be molten or at least be softened during continuous melt granulation. When molten or softened, the first edible excipient establishes bridges between the primary particles. Said bridges then solidify at room temperature. Therefore, the first edible excipient mostly acts as a binder. In the most preferred embodiment of the invention, the first edible excipient is sorbitol.

The melting temperature of a second edible excipient is relatively high such that - typically - it does not fully melt or does not melt at all during continuous melt granulation. The second edible excipient mostly acts as a filler. In the most preferred embodiment, the second edible excipient is inulin.

The granule of the present invention may comprise one kind of primary particles only or preferably more than one kind of primary particles. The primary particles of the granule of the invention are preferably water-soluble or water-dispersible and comprise preferably at least one active ingredient. In case of fat-soluble active ingredients, the primary particles are preferably water-soluble or water-dispersible microcapsules that encapsulate a fat-soluble active ingredient. Such microcapsules can be obtained by spray-drying of an emulsion comprising the lipophilic active ingredient and at least one emulsifier.

The granules of the present invention are preferably water-soluble or water-dispersible. Compositions comprising or consisting of such granules are suitable for preparing a beverage.

### Filler of the invention

Fillers are excipients used to increase the volume of the granule of the invention. However, fillers can have further functions. Some fillers (e.g. dietary fibers) also have health benefits.

The granule of the invention is meant for human consumption. Toxic fillers and non-edible fillers in general are therefore excluded.

The granule of the invention is preferably water-soluble or water-dispersible. Fillers having a solubility of less than 1 g per 100 mL water or less than 0.5 g per 100 mL water or less than 0.1 g per 100 mL water are therefore not preferred.

Typically, the melting temperature of the filler is higher than the melting temperature of the binder. There is no need to melt or soften the filler during continuous melt granulation. The melting temperature of the filler is preferably at least 150°C , more preferably at least 155°C and most preferably at least 160°C. The melting temperature of the filler is preferably from 151°C to 240° C, is more preferably from 180° C to 240°C and is most preferably from 180°C to 200° C.

Preferred fillers are inulin, human milk oligosaccharides (HMOs) and mannitol. 2'-fucosyllactose (2'-FL) is the preferred HMO. A preferred filler is a mixture comprising 2'-fucosyllactose (2'-FL) and difucosyllactose (DFL). Granulated standard inulin is the preferred inulin. Standard inulin has a mean degree of polymerization (DP) from 10 to 20. Not preferred fillers are microcrystalline cellulose and pregelatinized starch.

### Binder of the invention

Binders are excipients used to hold the ingredients of a formulation together (i.e. are solid at room temperature). To do so, the binder is melted or softened during continuous melt granulation. Typically, the melting temperature of the binder is lower than the melting temperature of the filler and is often also lower than the melting temperature of any added active ingredient. The melting temperature of the binder is preferably less than 140°C, more preferably less than 130°C, even more preferably less than 120°C, and most preferably less than 110°C. The melting temperature of the binder is preferably from 50°C to 110°C, is more preferably from 60°C to 100°C and is most preferably from 70°C to 100° C.

The granule of the invention is preferable water-soluble or water-dispersible. Binders having a solubility of less than 1 g per 100 mL water or less than 0.5 g per 100 mL water or less than 0.1 g per 100 mL water are therefore not preferred. Possible binders are *inter alia* ribose (such as D-ribose), polyethylene glycol, sorbitol and xylitol. The binder of the invention is preferably a polyol, is more preferably a sugar alcohol, is even more preferably sorbitol or ribose (e.g. D-ribose), and is most preferably sorbitol having a melting temperature of 98°C or less. Such sorbitol is commercially available at Roquette^{®}.

### Microcapsule of the invention

Microencapsulation is a protective technology for encapsulating solid or liquid active ingredients into microparticles with a diameter of e.g. 1-900 µm. The granule of the invention comprises multiple microcapsules. Thus, the size of the granule depends *inter alia* on the size of the microcapsules: relatively large microcapsules result in relatively large granules.

One advantage of microencapsulation lies in that the solid or liquid active ingredient is completely or almost completely coated and isolated from external environment. Microencapsulation may render fat-soluble active ingredients water-dispersible or water-soluble.

Spray-drying is a suitable method for the microencapsulation of fat-soluble active ingredients. Prior to spray-drying, the fat-soluble active ingredient is mixed with at least one encapsulating agent. Typically, an emulsion is thereby provided. Gum Arabic is a commonly used encapsulating agent. Spray-drying is one of the most widely used microencapsulation techniques, since it provides rapid evaporation of water and maintains the low temperature in the particles. Spray-dried microcapsules comprising fat-soluble vitamins are commercially available at DSM Nutritional Products, Switzerland. Vitamins A, D, E, and K are exemplary fat-soluble vitamins. This includes derivatives thereof such as esters.

It is rather uncommon to encapsulate a mixture of fat-soluble vitamins. Usually, fat-soluble vitamins are separately encapsulated. When providing a mixture of multiple fat-soluble vitamins, various kinds of microcapsules are mixed.

### Mixture of the invention

The mixture of the invention is suitable for continuous melt granulation. It is a powderous mixture that can be fed into an apparatus that is suitable for continuous melt granulation (e.g. an extruder).

In contrast to wet granulation, no solvent is needed when doing continuous melt granulation. Thus, the mixture of the invention comprises less than 10 weight-%, preferably less than 8 weight-%, more preferably less than 5 weight-% and most preferably less than 3 weight-% solvent, based on the total weight of the mixture. This applies not only, but in particular when the solvent is water. Thus, the preferred mixture of the invention comprises less than 10 weight-%, preferably less than 8 weight-%, more preferably less than 5 weight-% and most preferably less than 3 weight-% water, based on the total weight of the mixture.

The mixture of the invention comprises or consists of a filler, a binder and microcapsules.

Fillers are needed for size enlargement; they increase the volume of the granule of the invention. The mixture of the invention comprises preferably from 40 weight-% to 95 weight-%, more preferably from 60 weight-% to 90 weight-% and most preferably from 70 weight-% to 80 weight-% of at least one filler, based on the total weight of the mixture. The mixture of the invention may comprise more than one filler. Preferably, however, the invention may comprises one filler only. Thereby, the filler is preferably a polysaccharide, is more preferably a dietary fiber, is even more preferably inulin and is most preferably granulated inulin powder.

Typically, the mixture of the invention comprises less binder than filler. The weight ratio between the filler and the binder is preferably from 4:1 to 10:1, is more preferably from 5:1 to 10:1, is even more preferably from 6:1 to 9:1 and is most preferably from 7:1 to 8:1. The mixture of the invention comprises preferably from 5 weight-% to 15 weight-%, more preferably from 6 weight-% to 14 weight-% and most preferably from 8 weight-% to 13 weight-% of at least one binder, based on the total weight of the mixture. The mixture of the invention may comprise more than one binder. Preferably, however, the mixture of the invention comprises one binder only. Thereby, the binder is preferably a polyol, is more preferably a sugar alcohol, is even more preferably sorbitol or ribose, and is most preferably sorbitol. The preferred ribose is D-ribose.

The mixture of the invention comprises microcapsules. When granulating the mixture of invention, there is a certain risk that some of the microcapsules might be damaged. This risk can be reduced by creating a low-shear environment in the extruder (e.g. by choosing suitable kneading elements). However, this risk can also be reduced by decreasing the concentration of microcapsules in the mixture of the invention. The mixture of the invention comprises preferably from 0.1 weight-% to 55 weight-%, more preferably from 1 weight-% to 40 weight-%, even more preferably from 1 weight-% to 30 weight-% and most preferably from 5 weight-% to 20 weight-% microcapsules, based on the total weight of the mixture.

In an embodiment, the herein described microcapsules encapsulate at least one fat-soluble active ingredient. Preferred fat-soluble active ingredients are fat-soluble micronutrients such as fat-soluble vitamins and pro-vitamins. Beta-carotene is an example of a fat-soluble pro-vitamin. In one embodiment, the mixture of the invention comprises one kind of microcapsules only. In a preferred embodiment, however, the mixture of the invention comprises various kinds of microcapsules. In case of various kinds of microcapsules, the mixture of the invention comprises preferably at least two, more preferably at least three, even more preferably at least four and most preferably at least five fat-soluble active ingredients. In one embodiment, the mixture of the invention comprises microcapsules comprising vitamin A, microcapsules comprising vitamin E, microcapsules comprising beta-carotene, microcapsules comprising vitamin D and/or microcapsules comprising vitamin K. In a preferred embodiment, the mixture of the invention comprises microcapsules comprising an ester of vitamin A (such as vitamin A acetate), microcapsules comprising an ester of vitamin E (such as vitamin E acetate), microcapsules comprising beta-carotene, microcapsules comprising vitamin D3 and/or microcapsules comprising vitamin K1.

### Granules of the invention

Preferred granules are obtainable by continuous melt granulation (i.e. without solvent) of the mixture of the invention, preferably using a twin-screw extruder. Thus, the granule of the invention comprises or consists of the mixture of the invention.

The mixture of the invention comprises primary particles being preferably microcapsules. Upon continuous melt granulation, bridges are formed between the mixture's primary particles. Thus, the granule of the invention is larger than the size of its primary particles. Preferred granules of the present invention have a mass median particle size D50 (volume based) from 0.5 mm to 6 mm, preferably from 1 mm to 5 mm, more preferably from 1.5 mm to 4.5 mm and most preferably from 2 mm to 4 mm, measured using dynamic image analysis. Granules of the present invention may comprise more than 10, more than 100, more than 500 or even more than 1000 microcapsules. Each of the granule may comprise one kind of microcapsules only. In an embodiment, however, the granule of the invention comprises various kinds of microcapsules. In case of various kinds of microcapsules, the granule of the invention comprises preferably at least two, more preferably at least three, even more preferably at least four and most preferably at least five fat-soluble active ingredients. In one embodiment, the granule of the invention comprises microcapsules comprising vitamin A, microcapsules comprising vitamin E, microcapsules comprising beta-carotene, microcapsules comprising vitamin D, and/or microcapsules comprising vitamin K. In a preferred embodiment, the granule of the invention comprises microcapsules comprising an ester of vitamin A (such as vitamin A acetate), microcapsules comprising an ester of vitamin E (such as vitamin E acetate), microcapsules comprising beta-carotene, microcapsules comprising vitamin D3 and/or microcapsules comprising vitamin K1.

In one embodiment, the granule of the invention comprises a filler, a binder and microcapsules,
wherein the granule comprises from 0.1 weight-% to 55 weight-% of microcapsules, based on the total weight of the granule, and
wherein the granule comprises from 5 weight-% to 15 weight-% binder, based on the total weight of the granule, and
wherein the weight ratio between the filler and the binder is from 4:1 to 10:1, and
wherein the melting temperature of the binder is lower than the melting temperature of the filler, and
wherein the melting temperature of the filler is from 151°C to 240°C.

The granule of the present invention is preferably water-soluble or water-dispersible. This can be achieved by selecting a binder that is water-soluble or water-dispersible, by selecting a filler that water-soluble or water-dispersible, and by selecting microcapsules that water-soluble or water-dispersible.

### Method of the invention

The method of the invention is continuous melt granulation and is preferably continuous twin-screw melt granulation. Differences between batch melt granulation and continuous twin-screw melt granulation are listed in Table 1 of N. I<ittikunakorn et al., "Twin-screw melt granulation: Current progress and challenges", International Journal of Pharmaceutics, 588, (2020), 119670. In a preferred embodiment of the invention, the herein disclosed dry, powderous mixture is fed into an extruder that is suitable for continuous melt granulation. Volumetric powder feeders are thereby not preferred. In a preferred method of the invention, the mixture of the invention is fed into the herein described extruder using a gravimetric powder feeder. Gravimetric powder feeders obtain a controlled and consistent feeding process, keeping changes in powder properties and process deviations over time into consideration.

In the method of the invention, a twin-screw extruder is preferably being used. Particularly preferred are twin-screw extruders with corotating screws. The corotating screws of the preferred extruder are modular and can hence be configured in a variety of setups, resulting in various zones. The purpose of the first zone near the inlet of the extruder is transport. Transport zones are often referred to as conveying zones. One or more kneading zones can be present. The kneading zone is typically located between two conveying zones with preferably a shaping zone at the extruder outlet.

Most often, each zone has different screw elements. The conveying zone has conveying elements that transport material towards the granulator outlet [cf. section 2.1 of N. I<ittikunakorn et al., "Twin-screw melt granulation: Current progress and challenges", International Journal of Pharmaceutics, 588, (2020), 119670]. The kneading zone has kneading elements with narrower or wider kneading disks. A typical shaping zone has at least one size control element that minimizes the amount of oversized granules. An exemplary size control element is shown in Figure 1(f) of J. Vercruysse et al. "Impact of screw configuration on the particle size distribution of granules produced by twin screw granulation", International Journal of Pharmaceutics 479 (2015) 171-180. These size control elements are not knives as used for cutting extruded strands. Indeed, no spaghetti-like strands are extruded when doing continuous melt granulation. Extruders that are suitable for continuous melt granulation do not have a die at the outlet [cf. Fig. 1 of N. I<ittikunakorn et al., "Twin-screw melt granulation: Current progress and challenges", International Journal of Pharmaceutics, 588, (2020), 119670]. Size control elements are screw elements within the extruder.

Hot-melt extrusion is different from the herein described continuous melt granulation. When doing hot-melt extrusion, strands with e.g. a cylindric diameter are extruded through a die. The length of the strand is not limited (i.e. could be endless). To obtain separated units, strands obtained by hot-melt extrusion need to be cut into pieces. The obtained pellets are not granules formed of distinguishable primary particles. When doing hot-melt extrusion, the cutting step can be done at any time after extrusion, including directly at the die of the extruder. Dies with an integrated knife are commercially available.

The above does not apply to the method of the present invention. When doing continuous melt granulation, no strand is extruded. Instead, granules are continuously churned out at the end of the extruder. Because no strand is produced, there is no need for a knife/cutting step, which significantly simplifies the process. When doing continuous melt granulation, a die at the end of the extruder is not needed. In a preferred embodiment of the invention, the mixture of the invention is fed into a twin-screw extruder that has no die and no knife cutting device.

In the method of the invention, the screw configuration of a twin-screw extruder is typically selected such that the extruder has at least one kneading zone. Thereby, the at least one kneading zone is preferably closer to the powder inlet of the extruder than to the end of the extruder. I<neading zones have kneading elements. Said kneading elements are preferably kneading disks as disclosed in US 2005/0041521. Kneading disks may be congruent or non-congruent and are preferably positioned at a stagger angle from 30° to 90°. A stagger angle of approx. 30° is thereby preferred, as this is limiting the stress exerted on the powder mixture. In the context of the invention, "stagger angle" refers to the angle of crest misalignment that make two directly successive kneading disks, as explained in paragraph [0007] of US 2005/0041521. By way of example, the expression "kneading disks are positioned at a stagger angle 30°" means that there are successive kneading disks which make an angle of crest misalignment of 30°. There may be more than two successive kneading disks in a kneading zone of an extruder. Figure 2 of US 2005/0041521 is a lateral view of a kneading zone with five successive kneading disks, wherein the kneading disks are positioned at a predetermined stagger angle.

Temperature control is relevant when doing continuous melt granulation. In a preferred method of the invention, the extruder has several zones which can be heated up or cooled down individually. When continuously melt granulating the herein disclosed mixture, temperature zones closer to the powder inlet of the extruder are typically heated. When choosing a suitable temperature, it must be taken into consideration that the material in the extruder may be moving rather quickly such that the contact of the material with the heating element is rather short. In some cases, it may therefore be advisable to set the temperature of some zones of the extruder to a temperature above the melting temperature of the mixture's binder.

In a preferred embodiment of the invention, the conveying zone and/or the kneading zone of the extruder are heated, preferably to a temperature from 90°C to 200°C, more preferably to a temperature from 100°C to 180°C and most preferably to a temperature from 110°C to 170°C.

Churning out hot granules is not preferred. Hot granules may still be relatively soft and sticky. As a consequence, hot granules may form a lump. This is to be avoided. It is therefore preferred to cool the material in the extruder before it is churned out by the extruder. In a preferred embodiment of the invention, at least one of the zones after the kneading is cooled down to a temperature of less than 60°C, preferably less than 40°C and most preferably less than 26°C.

### Examples

### Example 1 (vitamin premix)

In example 1, a powderous premix comprising a mixture of microcapsules was provided. Each kind of microcapsule comprised a fat-soluble vitamin or a precursor of a fat-soluble vitamin. All microcapsules were obtained from DSM Nutritional Products (Switzerland).

The premix provided in example 1 comprised vitamin A acetate, beta-carotene, vitamin D3, vitamin E acetate and vitamin K.

### Example 2 (ribose as binder)

In example 2, a dry mixture comprising 47.13 weight-% inulin Orafti^{®}GR (filler), 10 weight-% D-ribose (binder) and 42.87 weight-% premix of example 1, based on the total weight of the dry mixture, was provided. Thus, the weight ratio between filler and binder was 4.7:1.

Pre-experiments showed that the lowest possible processing temperature of D-ribose, still generating granules with desirable quality attributes, is approx. 80° C.

Orafti^{®}GR is granulated inulin powder (average degree of polymerization ≥ 10), available at Beneo, Mannheim, Germany). Its melting point has been determined in the range 190-195°C.

The dry mixture of example 2 was then fed into a ThermoFisher^{®} Eurolab^{®} extruder, using a gravimetric loss-in-weight feeder at the powder inlet. The extruder had a length-to-diameter (L/D) ratio of 25/1 and a screw diameter of 16 mm. The corotating screws of the extruder were fully modular and could hence be configured in a variety of setups. The extruder was segmented in several zones which can be heated up or cooled down individually.

In example 2, five extrusion experiments were run using various temperature schemes. Temperature zones closer to the powder inlet (zones 2, 3 and 4) were heated to temperatures from 104°C to 130°C. The temperature of zones closer to the end of the extruder (zones 5 and 6) was kept at 25°C. Cooling the end of the extruder enables material solidification and prevents stickiness of the granules that are being churned out of the extruder.

In example 2, the extruder had one kneading zone with three wide kneading disks that were positioned at a stagger angle of 60° (i.e. the angle of crest misalignment between any two directly successive kneading disks made 60°C).

Reasonable granules were obtained in all five extrusion experiments. However, regardless of the applied temperature scheme, oil was oozing out of the obtained granules, indicating that some microcapsules were damaged during the extrusion process.

### Example 3 (sorbitol as binder)

In example 3, the process of example 2 was repeated. However, instead of D-ribose, sorbitol was used as binder. Pre-experiments showed that the lowest possible processing temperature of sorbitol, still generating granules with desirable quality attributes, was approx. 85°C (i.e. slightly higher than D-ribose).

In example 3, five extrusion experiments were run. Temperature schemes and screw configurations similar to example 2 were applied.

Reasonable granules were obtained in all five experiments. Less oil was oozing out of the granules, indicating that sorbitol is performing better than ribose.

Different grades of sorbitol were then investigated in order to further reduce the energy required to generate good water soluble granules. Samples were analyzed via differential scanning calorimetry (DSC) in order to determine the sorbitol grade with the lowest melt energy. When using sorbitol grade Xtab300S (available at Roquette^{®}) as a binder, the energy required to generate good water-soluble granules is lower than the other grades (cf. Table below).

| Sorbitol Grade | Melt temperature (C) | Melt Energy (J/g) |
|---|---|---|
| Xtab300S | 97.84 | 126.5 |
| Xtab200S | 97.81 | 161.7 |
| P550SD | 97.86 | 165.1 |
| P650C | 99.73 | 174.9 |
| P300C | 99.64 | 180.3 |
| P100C | 99.57 | 181.9 |

### Example 4 (more filler; effect of dilution)

In example 4, the process of example 3 was repeated. However, less premix and more filler was used. The dry mixture of example 4 comprised 72.85 weight-% inulin Orafti^{®} GR (filler), 10 weight-% sorbitol (binder) and 17.15 weight-% premix of example 1, based on the total weight of the dry mixture. Thus, the weight ratio between filler and binder was approx. 7.3:1.

In example 4, five extrusion experiments were run. Temperature schemes of example 2 were applied. A screw configuration consisting of a single kneading zone with 3 kneading disks positioned at a stagger angle of 30° was used.

Good quality granules were obtained in all five experiments. No oil was visually oozing out of the granules. Example 4 shows the benefit of diluting the microcapsules with filler: when reducing the concentration of microcapsules in the mixture that were fed into the extruder, less stress was exerted on the microcapsules. Example 4 also shows the benefit of using a stagger angle of less than 60°.

### Example 5 (alternative fillers)

In example 5, alternative fillers were tested. Several fillers were processed as a placebo blend (90% filler and 10% sorbitol as binder) in order to see which fillers are suitable for continuous melt extrusion. The trials showed that human milk oligosaccharides (2'-fucosyllactose; melting point approx. 230°C), mannitol (Pearlitol 160C; melting point approx. 165°C) and pregelatinized starch (Lycatab PGS; melting point approx. 257°C) are also suitable to manufacture granules for a processing time of more than one hour. However, granules formed with Lycatab PGS had a poor solubility. Therefore, pregelatinized starch is not preferred. Also not preferred is non-granulated inulin. The trial with Inulin HSI^{®} (a high soluble inulin powder) resulted in torque overload such that a processing time of more than one hour could not be achieved. Also not preferred is microcrystalline cellulose (melting point approx. 250°C). Avicel PH200 is dispersible in water but is not water-soluble.

## Claims

1. A mixture comprising a filler, a binder and microcapsules,
wherein the mixture comprises from 0.1 weight-% to 55 weight-% of microcapsules, based on the total weight of the mixture, and
wherein the mixture comprises from 5 weight-% to 15 weight-% binder, based on the total weight of the mixture, and
wherein the weight ratio between the filler and the binder is from 4:1 to 10:1, and
wherein the melting temperature of the binder is lower than the melting temperature of the filler, and
wherein the melting temperature of the filler is from 151°C to 240° C.

2. The mixture according to claim 1, wherein the filler is a polysaccharide, is preferably a dietary fibre, is more preferably inulin and is most preferably granulated inulin powder.

3. The mixture according to claim 1 or 2, wherein the binder is a polyol, is preferably a sugar alcohol, is more preferably sorbitol or ribose, and is most preferably sorbitol.

4. The mixture according to any one of claims 1-3, wherein the filler is a dietary fibre and the binder is a sugar alcohol.

5. The mixture according to any one of claims 1-4, wherein the filler is inulin and the binder is sorbitol or ribose.

6. The mixture according to any one of claims 1-5, wherein the weight ratio between the filler and the binder is from 5:1 to 10:1, is preferably from 6:1 to 9:1 and is most preferably from 7:1 to 8:1.

7. The mixture according to any one of claims 1-6, wherein the microcapsules encapsulate a fat-soluble active ingredient.

8. The mixture according to any one of claims 1-7, wherein the microcapsules encapsulate at least two, preferably at least three, more preferably at least four and most preferably at least five fat-soluble active ingredients, wherein the mixture comprises:
- microcapsules comprising vitamin A, and/or
- microcapsules comprising vitamin E, and/or
- microcapsules comprising beta-carotene, and/or
- microcapsules comprising vitamin D, and/or
- microcapsules comprising vitamin K.

9. The mixture according to any one of claims 1-8, wherein the mixture comprises less than 10 weight-% water, preferably less than 8 weight-% water, more preferably less than 5 weight-% water and most preferably less than 3 weight-% water, based on the total weight of the mixture.

10. Granules comprising the mixture according to any one of claims 1-9.

11. Granules consisting of the mixture according to any one of claims 1-9.

12. The granules according to claim 10 or 11, wherein the granules have a mass median particle size D50 (volume based) from 0.5 mm to 6 mm, preferably from 1 mm to 5 mm, more preferably from 1.5 mm to 4.5 mm and most preferably from 2 mm to 4 mm, measured using dynamic image analysis.

13. The granules according to any one of claims 10-12, wherein the granules are obtainable by continuous melt granulation of the mixture according to any one of claims 1-9, preferably using a twin-screw extruder.

14. The granules according to any one of claims 10-13, wherein the granules are water-soluble or water-dispersible.

15. Use of the mixture according to any one of claims 1-9 for continuous melt granulation.

16. A method of manufacturing granules comprising the step of feeding the mixture according to any one of claims 1-9 into an extruder.

17. The method according to claim 16, wherein the extruder is a twin-screw extruder that has no cutting device.

18. The method according to claim 16 or 17, wherein the method does not comprise the step of cutting an extruded strand.

19. The method according to any one of claims 16-18, wherein the extruder has at least one kneading zone, and wherein said at least one kneading zone is preferably heated, preferably to a temperature from 90°C to 150°C, more preferably to a temperature from 100°C to 150°C and most preferably to a temperature from 110°C to 140°C.

## Patentansprüche

1. Mischung, umfassend einen Füllstoff, ein Bindemittel und Mikrokapseln,
wobei die Mischung 0,1 Gew.-% bis 55 Gew.-% Mikrokapseln, bezogen auf das Gesamtgewicht der Mischung, umfasst und wobei die Mischung 5 Gew.-% bis 15 Gew.-% Bindemittel, bezogen auf das Gesamtgewicht der Mischung, umfasst und wobei das Gewichtsverhältnis von Füllstoff zu Bindemittel 4:1 bis 10:1 beträgt und
wobei die Schmelztemperatur des Bindemittels niedriger als die Schmelztemperatur des Füllstoffs ist und
wobei die Schmelztemperatur des Füllstoffs 151 °C bis 240 °C beträgt.

2. Mischung nach Anspruch 1, wobei es sich bei dem Füllstoff um ein Polysaccharid, vorzugsweise einen Ballaststoff, besonders bevorzugt Inulin und am meisten bevorzugt granuliertes Inulinpulver handelt.

3. Mischung nach Anspruch 1 oder 2, wobei es sich bei dem Bindemittel um ein Polyol, vorzugsweise einen Zuckeralkohol, besonders bevorzugt Sorbit oder Ribose und ganz besonders bevorzugt Sorbit handelt.

4. Mischung nach einem der Ansprüche 1-3, wobei es sich bei dem Füllstoff um einen Ballaststoff und bei dem Bindemittel um einen Zuckeralkohol handelt.

5. Mischung nach einem der Ansprüche 1-4, wobei es sich bei dem Füllstoff um Inulin und bei dem Bindemittel um Sorbit oder Ribose handelt.

6. Mischung nach einem der Ansprüche 1-5, wobei das Gewichtsverhältnis von Füllstoff zu Bindemittel 5:1 bis 10:1, vorzugsweise 6:1 bis 9:1 und am meisten bevorzugt 7:1 bis 8:1 beträgt.

7. Mischung nach einem der Ansprüche 1-6, wobei die Mikrokapseln einen fettlöslichen Wirkstoff einkapseln.

8. Mischung nach einem der Ansprüche 1-7, wobei die Mikrokapseln mindestens zwei, bevorzugt mindestens drei, besonders bevorzugt mindestens vier und am meisten bevorzugt mindestens fünf fettlösliche Wirkstoffe einkapseln, wobei die Mischung Folgendes umfasst:
- Mikrokapseln, die Vitamin A umfassen, und/oder
- Mikrokapseln, die Vitamin E umfassen, und/oder
- Mikrokapseln, die beta-Carotin umfassen, und/oder
- Mikrokapseln, die Vitamin D umfassen, und/oder
- Mikrokapseln, die Vitamin K umfassen.

9. Mischung nach einem der Ansprüche 1-8, wobei die Mischung weniger als 10 Gew.-% Wasser, vorzugsweise weniger als 8 Gew.-% Wasser, besonders bevorzugt weniger als 5 Gew.-% Wasser und am meisten bevorzugt weniger als 3 Gew.-% Wasser, bezogen auf das Gesamtgewicht der Mischung, umfasst.

10. Granulat, umfassend die Mischung nach einem der Ansprüche 1-9.

11. Granulat, bestehend aus der Mischung nach einem der Ansprüche 1-9.

12. Granulat nach Anspruch 10 oder 11, wobei das Granulat eine massenmediane Teilchengröße D50 (volumenbezogen) von 0,5 mm bis 6 mm, vorzugsweise 1 mm bis 5 mm, besonders bevorzugt 1,5 mm bis 4,5 mm und am meisten bevorzugt 2 mm bis 4 mm, gemessen mittels dynamischer Bildanalyse, aufweist.

13. Granulat nach einem der Ansprüche 10-12, wobei das Granulat durch kontinuierliche Schmelzgranulation der Mischung nach einem der Ansprüche 1-9, vorzugsweise unter Einsatz eines Doppelschneckenextruders, erhältlich ist.

14. Granulat nach einem der Ansprüche 10-13, wobei das Granulat wasserlöslich oder wasserdispergierbar ist.

15. Verwendung der Mischung nach einem der Ansprüche 1-9 zur kontinuierlichen Schmelzgranulation.

16. Verfahren zur Herstellung von Granulat, umfassend den Schritt des Einspeisens der Mischung nach einem der Ansprüche 1-9 in einen Extruder.

17. Verfahren nach Anspruch 16, wobei der Extruder ein Doppelschneckenextruder ohne Schneidvorrichtung ist.

18. Verfahren nach Anspruch 16 oder 17, wobei das Verfahren nicht den Schritt des Schneidens eines extrudierten Strangs umfasst.

19. Verfahren nach einem der Ansprüche 16-18, wobei der Extruder mindestens eine Knetzone aufweist und wobei die mindestens eine Knetzone vorzugsweise erhitzt wird, vorzugsweise auf eine Temperatur von 90 °C bis 150 °C, besonders bevorzugt auf eine Temperatur von 100 °C bis 150 °C und am meisten bevorzugt auf eine Temperatur von 110 °C bis 140 °C.

## Revendications

1. Mélange comprenant une charge, un liant et des microcapsules,
dans lequel le mélange comprend de 0,1 % en poids à 55 % en poids de microcapsules, par rapport au poids total du mélange, et
dans lequel le mélange comprend de 5 % en poids à 15 % en poids de liant, par rapport au poids total du mélange, et
dans lequel le rapport pondéral entre la charge et le liant est de 4:1 à 10:1, et
dans lequel la température de fusion du liant est inférieure à la température de fusion de la charge, et
dans lequel la température de fusion de la charge est de 151 °C à 240 °C.

2. Mélange selon la revendication 1, dans lequel la charge est un polysaccharide, est de préférence une fibre alimentaire, est plus préférablement de l'inuline et est le plus préférablement une poudre d'inuline granulée.

3. Mélange selon la revendication 1 ou 2, dans lequel le liant est un polyol, est de préférence un alcool de sucre, est plus préférablement le sorbitol ou le ribose, et est le plus préférablement le sorbitol.

4. Mélange selon l'une quelconque des revendications 1 à 3, dans lequel la charge est une fibre alimentaire et le liant est un alcool de sucre.

5. Mélange selon l'une quelconque des revendications 1 à 4, dans lequel la charge est l'inuline et le liant est le sorbitol ou le ribose.

6. Mélange selon l'une quelconque des revendications 1 à 5, dans lequel le rapport pondéral entre la charge et le liant est de 5:1 à 10:1, est de préférence de 6:1 à 9:1 et est le plus préférablement de 7:1 à 8:1.

7. Mélange selon l'une quelconque des revendications 1 à 6, dans lequel les microcapsules encapsulent un ingrédient actif liposoluble.

8. Mélange selon l'une quelconque des revendications 1 à 7, dans lequel les microcapsules encapsulent au moins deux, de préférence au moins trois, plus préférablement au moins quatre et le plus préférablement au moins cinq ingrédients actifs liposolubles, dans lequel le mélange comprend :
- des microcapsules comprenant de la vitamine A, et/ou
- des microcapsules comprenant de la vitamine E, et/ou
- des microcapsules comprenant du bêta-carotène, et/ou
- des microcapsules comprenant de la vitamine D, et/ou
- des microcapsules comprenant de la vitamine K.

9. Mélange selon l'une quelconque des revendications 1 à 8, dans lequel le mélange comprend moins de 10 % en poids d'eau, de préférence moins de 8 % en poids d'eau, plus préférablement moins de 5 % en poids d'eau et le plus préférablement moins de 3 % en poids d'eau, par rapport au poids total du mélange.

10. Granules comprenant le mélange selon l'une quelconque des revendications 1 à 9.

11. Granules constitués du mélange selon l'une quelconque des revendications 1 à 9.

12. Granules selon la revendication 10 ou 11, dans lesquels les granules ont une taille moyenne de particule en masse D50 (basée sur le volume) de 0,5 mm à 6 mm, de préférence de 1 mm à 5 mm, plus préférablement de 1,5 mm à 4,5 mm et le plus préférablement de 2 mm à 4 mm, mesurée en utilisant une analyse d'image dynamique.

13. Granules selon l'une quelconque des revendications 10 à 12, dans lesquels les granules peuvent être obtenus par granulation continue à l'état fondu du mélange selon l'une quelconque des revendications 1 à 9, de préférence en utilisant une extrudeuse à deux vis.

14. Granules selon l'une quelconque des revendications 10 à 13, dans lesquels les granules sont solubles dans l'eau ou dispersibles dans l'eau.

15. Utilisation du mélange selon l'une quelconque des revendications 1-9 pour une granulation continue à l'état fondu.

16. Procédé de fabrication de granules comprenant l'étape d'alimentation du mélange selon l'une quelconque des revendications 1-9 dans une extrudeuse.

17. Procédé selon la revendication 16, dans lequel l'extrudeuse est une extrudeuse à deux vis qui n'a pas de dispositif de coupe.

18. Procédé selon la revendication 16 ou 17, dans lequel le procédé ne comprend pas l'étape de découpe d'un brin extrudé.

19. Procédé selon l'une quelconque des revendications 16 à 18, dans lequel l'extrudeuse a au moins une zone de malaxage, et dans lequel ladite au moins une zone de malaxage est de préférence chauffée, de préférence jusqu'à une température de 90 °C à 150 °C, plus préférablement jusqu'à une température de 100 °C à 150 °C et le plus préférablement jusqu'à une température de 110 °C à 140 °C.
